# EUROPÄISCHE PATENTSCHRIFT

(11) **EP 2 024 740 B1**
(45) Veröffentlichungstag und Bekanntmachung des Hinweises auf die Patenterteilung: **26.01.2011**
(21) Anmeldenummer: 07725878.8
(22) Anmeldetag: 01.06.2007
(51) Int. Cl.: G01N 29/06, G01N 29/07, G01N 29/11, G01N 29/34, G01N 29/44, G01N 33/38, G01S 15/52, G01S 15/89

(54) **VERFAHREN ZUM DETEKTIEREN UND KLASSIFIZIEREN VON FEHLSTELLEN IN BAUTEILEN MITTELS ULTRASCHALL**
METHOD FOR DETECTION AND CLASSIFICATION OF FLAWS IN COMPONENTS BY MEANS OF ULTRASOUND
PROCÉDÉ DE DÉTECTION ET DE CLASSIFICATION DES DEFAUTS DE COMPOSANTS AU MOYEN DES ULTRASONS

(30) Priorität: 02.06.2006 DE 102006027132
(43) Veröffentlichungstag der Anmeldung: 18.02.2009
(73) Patentinhaber: Bundesanstalt für Materialforschung und -prüfung (BAM), 12205 Berlin (DE); Universität Kassel, 34125 Kassel (DE)
(72) Erfinder: MILMANN, Boris, 12107 Berlin (DE); KRAUSE, Martin, 10963 Berlin (DE); MIELENTZ, Frank, 14163 Berlin (DE); MAYER, Klaus, 34246 Kassel (DE)
(74) Vertreter: Pfenning, Meinig & Partner GbR
(86) Internationale Anmeldenummer: PCT/EP2007/005029
(87) Internationale Veröffentlichungsnummer: WO 2007/141015

(56) Entgegenhaltungen:
- WO-A-01/86281
- WO-A-82/02781
- US-A- 4 597 292
- SCHICKERT MARTIN ET AL: "Ultrasonic imaging of concrete elements using reconstruction by synthetic aperture focusing technique" JOURNAL OF MATERIALS IN CIVIL ENGINEERING, AMERICAN SOCIETY OF CIVIL ENGINEERS, MATERIALS ENGINEERING DIV, US, Bd. 15, Nr. 3, 1. Juni 2003 (2003-06-01), Seiten 235-246, XP009088756 ISSN: 0899-1561 in der Anmeldung erwähnt
- OTTO KROGGEL, JAN SCHERZER, REINHARD JANSOHN: "THE DETECTABILITY OF IMPROPER FILLED DUCTS WITH ULTRASOUND REFLECTION TECHNIQUES" NDT.NET, [Online] Bd. 7, März 2003 (2003-03), XP002448775 Gefunden im Internet: URL:http://www.ultrasonic.de/article/v07n0 3/kroggel/kroggel.htm> [gefunden am 2007-08-30] in der Anmeldung erwähnt
- MARTIN KRAUSE, FRANK MIELENTZ, BORIS MILMANN, DOREEN STREICHER: "ULTRASONIC IMAGING OF CONCRETE ELEMENTS: STATE OF THE ART USING 2D SYNTHETIC APERTURE" NDT-CE 2003, [Online] 2003, XP002448776 Gefunden im Internet: URL:http://www.ndt.net/article/ndtce03/pap ers/v051/v051.htm> [gefunden am 2007-08-30] in der Anmeldung erwähnt

## Beschreibung

Die Erfindung betrifft ein Verfahren zum Detektieren und Klassifizieren von Fehlstellen in Bauteilen, insbesondere von Verpressfehlern in Spannkanälen oder von Verdichtungsmängeln in Betonbauteilen nach dem Oberbegriff des Hauptanspruchs.

Es ist bekannt Ultraschall-Prüfverfahren bei einer Vielzahl von unterschiedlichen Bauteilen anzuwenden. Unter anderen werden auch Strukturen in Bauwerken, z. B. aus Beton oder ähnlichen Materialien geprüft. Viele Betonbauwerke der Gegenwart sind sog. Spannbetonbauten. Die Eigenschaft von Beton, nur eine relativ geringe Zugfestigkeit zu besitzen, wird durch eine künstliche innere Vorspannung der Objekte durch Stahlseile bzw. Spanndrähte erreicht. Die Stahlseile werden in sog. Spannkanälen oder auch Hüllrohren geführt und nach der Erhärtung des Betons gespannt. Danach werden die Hüllrohre mit Verpressmörtel verfüllt, um einen festen Verbund zwischen den Stahlseilen und der Betonkonstruktion zu erreichen. Dabei ist es wichtig, dass der Verpressmörtel die Hüllrohre vollständig ausfüllt und die Spanndrähte lückenlos ummantelt, da sich in Hohlräumen Feuchtigkeit ansammeln kann und die Spanndrähte durch Korrosion angegriffen und zerstört werden können. Solche Hohlräume bzw. Verpressfehler in Spannkanälen stellen einen Qualitätsmangel dar, der im Extremfall zum Versagen des Bauteils und zum Einstürzen des Bauwerks führt. Zur Beurteilung der Standsicherheit einer Betonkonstruktion ist ebenso die Kenntnis über Verdichtungsmängel und Kiesnester wesentlich.

Zur Detektion von Fehlstellen in Bauteilen ist auch das Ultraschallecho-Verfahren bekannt, bei dem Ultraschallwellen in die Oberfläche des zu untersuchenden Objektes eingestrahlt werden und die reflektierten Schallwellen erfasst werden. Dabei kommt es zu Streuvorgängen und abhängig von der Intensität der Reflexion wird auf Fehlstellen geschlossen. Die Detektion von Spannkanälen beruht auf der Intensität der Reflexion von der der Messoberfläche zugewandten Hüllrohrseite. Bei Lufteinschlüssen ist diese im Vergleich zu gut verpressten Bereichen signifikant intensiver (s. Krause, M., Mielentz, F, Milmann, B., Streicher, D., Müller, W., Ultrasonic imaging of concrete elements: State of the art using 2D synthetic aperture, in: DGZfP (Ed.): International Symposium of Nondestructive Testing in Civil engineering (NDT-CE) in Berlin, Germany, September 16-19, 2003, Proceedings on BB 85-CD, V51, Berlin (2003);
Kroggel, O.; Scherzer, J.; Jansohn, R.: The Detectability Of Improper Filed Ducts With Ultra-sound Reflection Techniques. NDT.net - March 2002, Vol. 7 No. 03; Schickert, M.; Krause, M.; Müller, W.: Ultrasonic Imaging of Concrete Elements using SAFT Reconstruction, Journal of Materials in Civil Engineering 15 (2003) 3, S. 235-246). Zusätzlich kann die Rückseitenreflexion des Hüllrohres zur Interpretation des Verpresszustandes herangezogen werden, die nur in gut verpressten Abschnitten auftritt.

WO 01/86281 A1 beschreibt ein akustisches Mikroskop, bei dem Ultraschallwellen in eine Probe eingebracht und die reflektierenden Schallwellen erfasst werden. Aus den Empfangssignalen werden B- und C-Abtastbilder erzeugt.

Aus der US 4 597 292 ist ein Verfahren zur Ultraschallmessung bekannt, bei dem Ultraschallwellen mit einer Mehrzahl von Frequenzen in einen Gegenstand eingestrahlt werden und anhand der rückgestrahlten Echosignale ein B-Verteilungsbild erhalten wird, mit dem Positionsinformationen bezüglich einer im Gegenstand vorhandenen interessierenden Stelle bestimmt werden.

Der Erfindung liegt die Aufgabe zugrunde, ein Verfahren zu Detektion und Klassifikation von Fehlstellen in Bauteilen zu schaffen, das gegenüber dem Ultraschallecho-Verfahren nach dem Stand der Technik die Aussagesicherheit bei der Feststellung von Fehlstellen verbessert.

Diese Aufgabe wird erfindungsgemäß durch die kennzeichnenden Merkmale des Hauptanspruchs in Verbindung mit den Merkmalen des Oberbegriffs gelöst.

Dadurch, dass an einer Mehrzahl von Stellen impulsförmige Ultraschallwellen in das Betonbauteil eingestrahlt werden und die reflektierten Ultraschallwellen gleichfalls an mehreren Stellen der Oberfläche aufgenommen werden und anschließend die hochfrequenten elektrischen Empfangssignale unter Heranziehung der Positionen der Stellen der Einstrahlung und Aufnahme zur Erzeugung einer dreidimensionalen Ortsverteilung der Streueigenschaften des Objektes analysiert und ausgewertet werden, wobei zusätzlich zur Amplitudeninformation der Phasenwert des Streuvorganges ausgewertet wird und die Phaseninformation der dreidimensionalen Ortsverteilung der Streueigenschaften des Objekts zugeordnet wird und wobei zur Ortung der Fehlstellen die Amplitudeninformation und zur Klassifizierung der Fehlstellen (unschädliche Streuanzeigen oder wirkliche schädliche Fehlstellen) die Amplituden und die Phaseninformation der dreidimensionalen Ortsverteilung herangezogen wird, wird die Signifikanz und Aussagesicherheit bei der Feststellung von Fehlstellen, z.B. Verpressfehler deutlich verbessert.

Es wird somit die Phasenlage zusammen mit der Amplitudeninformation des Streuvorgangs für die Charakterisierung des Zustandes des Bauteils beispielsweise des Spannkanals herangezogen. Es werden die Amplituden- und Phaseninformationen aus dem Ergebnis von Rekonstruktionen ausgewertet, da dabei die Messdaten auf den Streuvorgang besser fokussiert werden und eine räumliche Trennung verschiedener Streuvorgänge erreicht wird. Diese Auswertung kann manuell durch Analyse der graphischen Darstellung der Schnitte und Projektionen aus der dreidimensionalen Ortsverteilung der 2D- bzw. 3D-SAFT-Rekonstruktion in Form von vorzeichenbehafteten B-Bildern und C-Bildern oder automatisiert durch Berechnung des jeweils lokalen Phasenwertes erfolgen.

Auf diese Weise gelingt die Klassifizierung des Reflektors z.B. des unverfüllten Hüllrohrs, weil der Unterschied des Phasenwertes zwischen dem akustisch dichterem Reflektor, z.B. Stahl, und dem Verpressfehler, z.B. Luft, ausgewertet wird. Ein solches Verfahren kann auch zur Erkennung von verdichtungsmängeln und Kiesnestern im Beton angewendet werden, wobei durch die Auswertung der Phasenlage diese von Bewehrungsstäben unterschieden werden können.

Das erfindungsgemäße Verfahren erlaubt eine automatisierte Datenaufnahme, Auswertung und Dokumentation, wobei Rohdaten, Rekonstruktionen, Geometrieinformationen und Phasenbewertung objektbezogen ermittelt, visualisiert und gespeichert werden.

Im Folgenden wird das erfindungsgemäße Verfahren unter Heranziehung der beigefügten Figuren näher erläutert. Es zeigen:
- Fig.1: eine perspektivische schematische An- sicht eines Betonbauteils als Probe- körper,
- Fig. 2: eine Darstellung eines Ausschnitts aus den Messdaten vor der Rekonstruktion,
- Fig. 3: eine Amplituden- und Phasendarstellung der dreidimensionalen Rekonstruktion der Messdaten im Tiefenschnitt,
- Fig. 4: eine Amplituden- und Phasendarstellung der dreidimensionalen Rekonstruktion der Messdaten im Schnitt parallel zur Messfläche,
- Fig. 5: ein Konstruktionsplan eines weiteren Beispiels eines Betonbauteils mit ein- betonierten Metall- und Styrodurplat- ten,
- Fig. 6: eine Rekonstruktion der Messdaten hin- sichtlich der Amplitude als Schnitt in der Tiefe der Metallplatten und
- Fig. 7: eine Rekonstruktion der Messdaten hin- sichtlich der Phase als Schnitt in der Tiefe der Metallplatten.

Bei dem erfindungsgemäßen Verfahren, das an Hand von Betonbauteilen erläutert wird, werden Ultraschallwellen über geeignete Ultraschallwandler in das Betonbauteil eingekoppelt und die an der Rückseite des Betonbauteils oder an Fehlstellen, Bewehrungen und anderen Materialsprüngen reflektierten Ultraschallwellen werden von den Ultraschallwandlern empfangen. Geeignete Ultraschallwandler sind beispielsweise solche auf Piezobasis arbeitende Schwinger, die kein Koppelmittel benötigen. Es werden beispielsweise mehrere in einem Array angeordnete Einzelschwinger verwendet, die federnd gelagerte Kontaktspitzen aufweisen. Dabei kann einer oder mehrere der Einzelschwinger als Sender arbeiten, während die anderen als Empfänger dienen. Die Empfangssignale werden in verarbeitbare digitale Daten umgewandelt und gespeichert. Um das gesamte Bauteil untersuchen zu können, wird die Ultraschallmessung in einem dichten Raster auf der zugänglichen Oberfläche des Bauteils durchgeführt. Dabei muss darauf geachtet werden, dass die Messdaten qualitativ hochwertig sind. Außerdem werden die Sende- und Empfangspositionen der Ultraschallwandler oder - sensoren aufgezeichnet und gespeichert.

Im bekannten Stand der Technik werden die erfassten Rohdaten, die hinsichtlich der Amplitude der empfangenen Ultraschallwellen aussagekräftig sind, zusammen mit den Positionsangaben der Ultraschallsensoren mit einem 3D-Abbildungs- oder Imaging-Verfahren, wie einem 3D-SAFT-Algorithmus (Synthetic Aperture Focussing Technique) ausgewertet, wodurch das Volumen des Bauteils dreidimensional rekonstruiert und wodurch das Innere des Betonbauteils akustisch abgebildet und die Ortsverteilung der Streueigenschaften dargestellt wird.

Das Auffinden von Verpressfehlern in Spannkanälen als konkretes Ausführungsbeispiel beruht somit u.a. auf der Messung eines Intensitätsunterschiedes der Reflexion von Ultraschallimpulsen an den Spanngliedern, wobei an Lufteinschlüssen, die Verpressfehler darstellen, quasi eine Totalreflexion stattfindet, d.h. der Reflexionskoeffizient nimmt seinen maximal möglichen Wert an. Dem gegenüber ist die Reflexion an gut verpressten Hüllrohren geringer, da Teile des Schalls durch die dünne Hüllrohrwand und den Verpressmörtel in das Hüllrohr eindringen.

Für die Auswertung können somit aus der dreidimensionalen Rekonstruktion der Streueigenschaften Schnitte und Projektionen hergeleitet werden, die als B-bilder und als C-Bilder bekannt sind, wobei die C-Bilder parallel zur Einstrahloberfläche (parallel zur X- Y-Ebene) liegen, während die B-Bilder Schnittebenen durch das Material in Richtung der Einstrahlung sind.

An diesen Bildern kann die Tiefen- und Intensitätsverteilung der Reflexion abgelesen werden, aus der wiederum festgestellt werden kann, wo Fehler vorhanden sind. Aus Standardrekonstruktionen werden in einem wählbaren Tiefenraster die Intensitätsverteilungen geliefert. Dann werden die Koordinaten ausgewählt, in denen ein Reflektor entdeckt oder erwartet wird. Insgesamt ist der Visualisierungsvorgang interaktiv.

Zur sicheren Ortung von Verpressfehlern ist ein ausreichend deutlicher Unterschied in der Reflexion zwischen gut verpresstem und luftgefülltem Bereich erforderlich. In der Praxis wird das nicht immer erreicht, da die Ultraschallausbreitung zusätzlich durch die schlaffe Bewehrung, die Güte der Schallübertragung an der Betonoberfläche und den Zustand des Betons beeinflusst wird. Daher wird entsprechend der Erfindung für die Klassifizierung zusätzlich zur Amplitudeninformation, d.h. zu den Intensitätsunterschieden der Reflexion von Ultraschallimpulsen innerhalb des Bauteils, die durch die Reflexion bewirkte Phasenänderung der Ultraschallimpulse herangezogen, d.h. es wird die Phase des reflektierten Signals betrachtet.

Es ist bekannt, dass ein mit einem Piezowandler gemessener Ultraschallimpuls (Schalldruck) bei einer Reflexion an einem akustisch dichteren Material seine Impulsform beibehält, während sich bei der Reflexion an einer Grenzschicht zu einem dünneren Material ein Phasensprung von 180°, also eine Impulsumkehr ergibt, d.h. bei einer Reflexion an Stahl bleibt die Phase erhalten, während an Luft der Phasensprung auftritt.

Da in Betonbauteilen sich die Reflexionen von verschiedenen Grenzflächen überlagern, wobei im Ausführungsbeispiel der Übergang von Hüllrohrwand bzw. Stahllitze zum Verpressmörtel jeweils eine zusätzliche Grenzschicht darstellt, ist es erforderlich, nicht nur den Phasensprung des reflektierten Impulses zu betrachten, sondern die Phasendrehung, die sich insgesamt in einem mehrschichtigen System ergibt. Die oben skizzierten Fälle 0° und 180° Phasenlage sind Spezialfälle, die sich bei Impulsen und sinus- oder kosinusförmigen Signalen leicht verstehen lassen, da eine Phasendrehung von 180° bei Kosinus-Funktionen eine Vorzeichenumkehr bedeutet: cos(phi)= - cos(phi + 180°). Ein impulsförmiges Signal lässt sich in Spektralanteile zerlegen, die sich im Falle der Ultraschallanregung um eine Mittenfrequenz anordnen (Bandpass-Signal), wobei jeder Spektralanteil aus einer phasenverschobenen Kosinus-Funktion besteht. Dabei ist eigentlich das Spektrum kontinuierlich, d.h. es gibt alle Frequenzen, aber bei einer numerischen Spektralzerlegung (Diskrete Fouriertransformation DFT) werden diskrete Spektrallinien erhalten. Wird der Einfachheit halber nur die Spektrallinie bei der Mittenfrequenz des Prüfkopfes betrachtet, so gehört dazu eine Kosinus-Schwingung mit einer Amplitude und einer Phasenlage. Die Phasenlage bezieht sich jedoch auf den Anfangszeitpunkt der Spektralanalyse und dieser ist frei wählbar. Daher sind die Phasenlage der Spektrallinie und damit die Phasenlage des analysierten Impulses nur bis auf einen Offset bestimmbar. Da die Phasenlage für den Streuvorgang bestimmt werden muss, darf nicht der Startzeitpunkt des Sendesignals als Referenzpunkt verwendet werden, sondern einen typischer Zeitpunkt im empfangenen Streuimpuls. Bei einem symmetrischen Impuls kann man sich leicht auf die Impulsspitze "setzen" und identifiziert dann die Spitze als positiv oder negativ und damit auf 0° oder 180° Phasenlage.

Dagegen ist bei Impulsen, die durch Streuung an nicht ebenen Flächen, oder an Schichten entstehen, stets eine Überlagerung von vielen Impulsen vorhanden (bei nur einer Frequenz würde von Interferenz gesprochen), die die Symmetrie des Gesamtimpulses verändern, und damit wird die Festlegung des Referenzpunktes nicht mehr offensichtlich und die Analyse der Phasenlage wird willkürlich.

Im Folgenden wird der Unterschied zwischen Signalauswertung und Rekonstruktion zum besseren Verständnis dargestellt. Die obigen Aussagen bezogen sich auf das gemessene Ultraschallsignal. Abbildungsverfahren wie 3D-SAFT, die von einer einseitigen Messfläche ausgehen, übertragen die Phaseninformation (mangels begrenzten Auflösungsvermögens) auf die Rekonstruktion und die Signale werden ersetzt durch synthetisch fokussierte B-Bilder. Genauer gesagt, die Zeitkoordinate bei dem Signal wird ersetzt durch die Tiefenkoordinate in der Rekonstruktion (im Ausführungsbeispiel die z-Koordinate). Der angezeigte Phasenwert ist in beiden Fällen nicht identisch, hat aber die gleiche Tendenz. Dies ergibt sich aus der komplexen Struktur des SAFT Algorithmus, der einige Ursachen der Phasendrehung im Streusignal teilweise korrigiert (streugeometrieabhängige Anteile) andere aber nicht (dünne Schichten, Mehrfachreflexionen). Die Auflösung in der Rekonstruktion ist in Tiefenrichtung im Wesentlichen identisch mit der Auflösung der Zeitsignale, nur ist der Weg (Wellenlänge) mit Hilfe der Ausbreitungsgeschwindigkeit umzunormieren. Jedoch ist das fokussierte B-Bild der Rekonstruktion wesentlich rauschfreier als die Daten, daher funktioniert die Phasenbestimmung besser.

Mittels eines Algorithmus wird auch die Phase der reflektierten Ultraschallimpulse ausgewertet und eine dreidimensionale Rekonstruktion auf der Grundlage der Phaseninformation berechnet. Aus dem Ergebnis ergibt sich die Phasendrehung bei der Reflexion in einer Farbskala entsprechend den B- bzw. C-Bildern, wodurch es möglich ist, den Wert der Phasendrehung ortsaufgelöst abzulesen.

Zum gegenwärtigen Zeitpunkt wird zur Bestimmung der Phasenlage des Streuvorgangs folgendermaßen vorgegangen :
Phasenbestimmung aus der Impulsform der Rekonstruktion:

### Methode a):

Es wird die Tiefenskala aus den Amplituden-Abbildungen verwendet und der Signalverlauf in der Tiefe eines erwarteten Streuvorgangs (Oberkante Hüllrohr o.ä.) betrachtet und dann das Vorzeichen des Hauptimpulses erkannt.

### Methode b):

Es wird eine mathematische Einhüllende über das B-Bild (oder das Signal) - dies ist eine Art Intensitätswertbildung - berechnet, auf diese Art das Zentrum der Anzeige gefunden und an dieser Stelle das Vorzeichen des (nicht "eingehüllten") Original-B-Bildes analysiert.

### Rechnerische Methode:

Es wird die Einhüllende des B-Bildes (oder des Signals) berechnet und das Maximum der Einhüllenden als Phasen-Referenz-Punkt definiert. Von diesem Punkt aus wird der Impuls eine Schwingung der Mittenfrequenz nach rechts und nach links freigeschnitten und über eine Fouriertransformation dieses Ausschnitts der Phasenwert der Spektrallinie bei der Mittenfrequenz bestimmt, der nun Werte von 0° - 360° annehmen kann.

Dieser Wert wird dem jeweiligen Streuzentrum zugeordnet. Es werden also nicht Phasenwerte für das gesamte B-Bild, sondern nur für Streuzentren erhalten. Die Auswahl der Streuzentren erfolgt über einen Schwellenwert der Einhüllenden, der einstellbar ist.

Die Berechnung wurde grob oben geschildert; als Parameter gehen ein: Wellenlänge bei der Mittenfrequenz, zwei Schnittparameter für die Breite des auszuschneidenden Signalfensters sowie der Schwellwert der Identifikationsempfindlichkeit. Es wird in der aktuellen Implementierung die Phaseninformation für die gesamte Rekonstruktion berechnet und danach können die Schnitte zusammen mit der Amplitudeninformation interaktiv dargestellt werden. Um den geometrischen Zusammenhang in einem Bild leichter zu erfassen, ist im Phasenbild das Amplitudenbild als Schwarzweißbild für Werte kleiner als die Identifikationsschwelle mit angezeigt. Dazu wird die Phaseninformation als Farbwert, der die Amplitude als Helligkeitswert mit enthält, dargestellt. Mit Hilfe von Cursoren kann der Phasenwert als Zahlenwert an jedem Punkt der Rekonstruktion angezeigt werden.

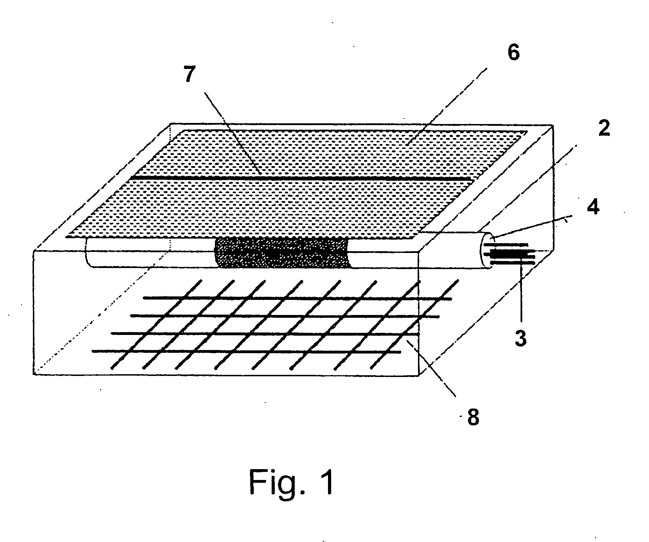

Fig. 1 zeigt die schematische Darstellung eines Betonbauteils 1 als Probekörper mit einem Hüllrohr 2, in das Stahllitzen 3 eingezogen und mittels Verpressmörtel 4 festgelegt sind. Ein Verpressfehler 5, d.h. ein unverpresster Bereich ist schraffiert dargestellt. In der Figur oben ist die Messfläche und insbesondere eine Messlinie 7 über dem Hüllrohr 2 auf dem Betonbauteil 1 und unten in demselben eine schlaffe Bewehrung 8 angeordnet. Im realen Bauteil ist üblicherweise eine Bewehrungslage auch zwischen Hüllrohr 2 und Messfläche 6 zu finden, was hier jedoch nicht berücksichtigt ist.

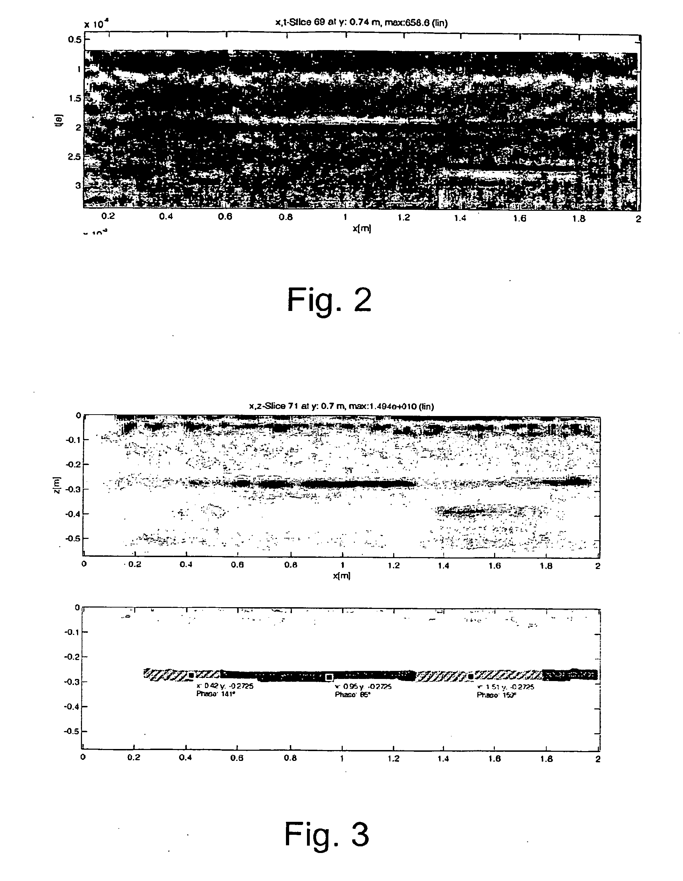

In den Fign. 2 bis 4 ist die Auswertung der in den Probekörper eingestrahlten und reflektierten Ultraschallwellen dargestellt.

Fig. 2 zeigt einen Ausschnitt (B-Bild) der flächigen Messung entlang einer Linie über dem Hüllrohr 2 in x-Richtung für y - 0,67 m, d.h. die Zeit t x 10⁴ s über dem Ort x in m. Aus dem Verlauf der Amplitude der Messsignale ist zu erkennen, dass eine Impulsverformung in einem Bereich des Hüllrohrs 2 auftritt, der als unverpresst gefertigt wurde. Es ist zwar auch eine Amplitudenänderung zu erkennen, die aber ebenso von der schlaffen Bewehrung zwischen Hüllrohr 2 und Messfläche herrühren könnte (tatsächlich existiert ein solcher Bereich im Probekörper).

Fig. 3 zeigt oben die Amplituden- und unten die Phasenauswertung der 3D-FT-SAFT Rekonstruktion der Messdaten nach Fig. 2 als B-Bild, d.h. als Tiefenschnitt x, z bei y = 0,7 m. Die Analyse der Daten zeigt, dass die Phase deutlich unterschiedliche Werte zwischen verpresstem und unverpresstem Bereich (ca. 150° gegenüber 86°) liefert. Da die Fign. nicht als Farbbilder wiedergegeben werden können, sind sie als schwarz/weiß Bilder mit zusätzlichen Schraffuren der relevanten Anzeigen dargestellt und beschriftet.

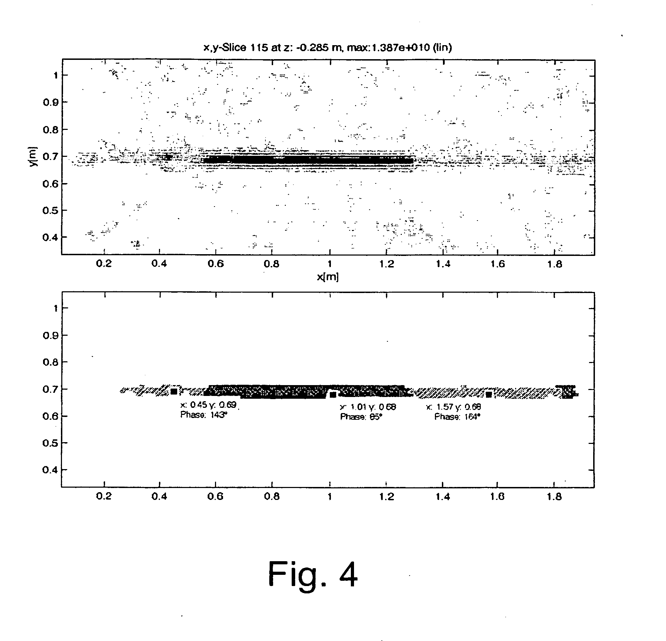

Fig. 4 zeigt eine Scheibe (C-Bild; x, y) der 3D-FT-SAFT Rekonstruktion der Messdaten der Fig. 2 hinsichtlich der Auswertung der Amplitude (oben) und der Phase (unten) parallel zur Messfläche in der Tiefe der Oberkante des Hüllrohrs 2 (z=-0,285m). Auch hier ist der nicht verpresste Bereich gut erkennbar.

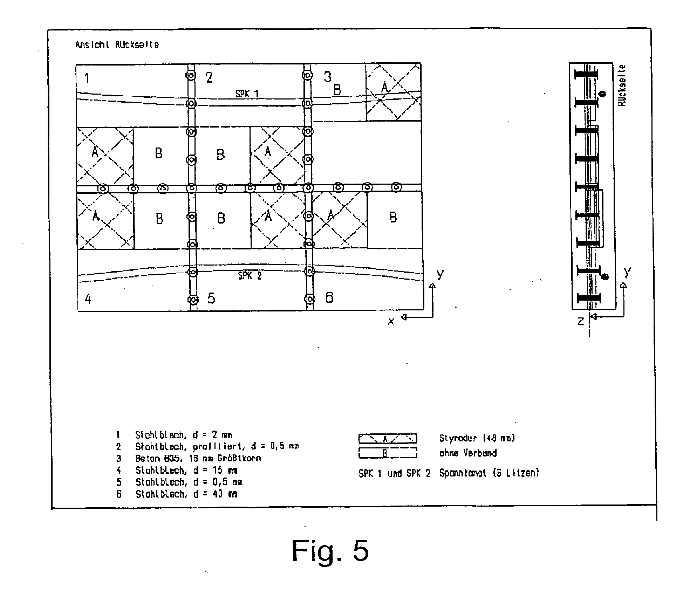

In Fig. 5 ist ein weiteres Beispiel eines Probekörpers, nämlich eines Plattenprüfkörpers von der Rückseite und im Schnitt dargestellt, der mehrere Metallplatten 1,2 und 4 bis 6 unterschiedlicher Dicke aufweist, die unter Anlegung von Styrodur A, das Luft darstellen soll, in Beton eingegossen sind. Das Feld 3 ist mit Beton und Styrodur ohne Metall ausgefüllt. Die Stellen ohne Styrodurverbund sind mit B bezeichnet. SPK1 und SPK2 sind Spannkanäle, die hinter den Metallplatten liegen.

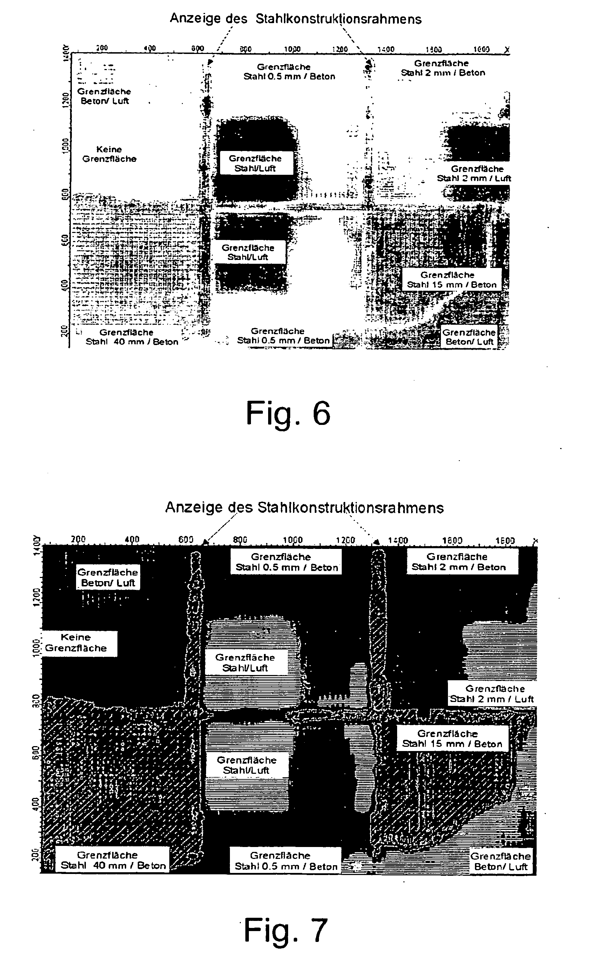

Die in den Fign. 6 und 7 angegebenen Rekonstruktionen sind Schnitte in einer Tiefe von etwa 10 cm, d.h. in der Tiefe der Oberkanten der Metallplatten von vorn gesehen. Die Styrodurplatten A befinden sich unter den Metallplatten und abhängig von den jeweiligen Dicken derselben in verschiedenen Tiefen. Wegen der Auflösung der Rekonstruktion sind Styrodurplatten A hinter den dünnen Blechen (Metallplatten) in der Ebene derselben zu sehen. Bei dicken Metallplatten sind sie jedoch außerhalb des dargestellten Tiefenbereichs. Die Styrodurplatte im Bereich 3 ohne Metallplatte ist bei der Fertigung aus der Lage verrutscht und befindet sich ebenfalls außerhalb des angezeigten Bereichs. Fig. 6 ist eine SAFT Rekonstruktion von Messdaten hinsichtlich der Amplitude an dem Betonteil nach Fig. 5. In der Auswertung sind deutlich Echoanzeigen von Grenzflächen erkennbar. Genauere Aussagen über die Art der Grenzfläche, wie fest/fest oder fest/gasförmig (hier also Beton/Stahl oder Stahl/Lufteinschluss) können nicht getroffen werden. Fig. 7 ist die Rekonstruktion hinsichtlich der Phase, wobei die Phase der reflektierten Impulse nun Aussagen über die Art der Grenzflächen ermöglicht. Dabei beruht die visuelle Erkennung auf einer Darstellung von unterschiedlichen Farben, die hier in schwarz/weiß bzw. unterschiedlichen Grauwerten vorgesehen ist.

## Patentansprüche

1. Verfahren zum Detektieren und Klassifizieren von Fehlstellen, Verdichtungsmängeln und Kiesnestern in Betonbauteilen (1) und/oder Verpressfehlern in Spannkanälen von Betonbauteilen mit folgenden Schritten:
Einstrahlen von impulsförmigen Ultraschallwellen aus elektrischen Sendeimpulsen an mehreren Stellen der Oberfläche des zu untersuchenden Bauteils (1),
Aufnehmen der reflektierten Ultraschallwellen an mehreren Stellen der Oberfläche zur Erzeugung einer Mehrzahl von elektrischen Empfangssignalen,
Analysieren und Auswerten der hochfrequenten elektrischen Empfangssignale unter Heranziehung der Positionen der Stellen der eingestrahlten Ultraschallwellen und der aufgenommenen reflektierten Schallwellen zur Erzeugung einer dreidimensionalen Ortsverteilung der Streueigenschaften des Bauteils (1),
**dadurch gekennzeichnet,**
**dass** zusätzlich zur Amplitudeninformation die Phaseninformation des Streuvorgangs ermittelt und der dreidimensionalen Ortsverteilung der Streueigenschaften des Bauteils (1) zugeordnet wird, wobei zur Detektion der Fehlstellen die Amplitudeninformation und zur Klassifikation die Amplitudeninformation und die Phaseninformation der dreidimensionalen Ortsverteilung herangezogen wird.

2. Verfahren nach Anspruch 1, **dadurch gekennzeichnet, dass** das Einstrahlen und Aufnehmen von Ultraschallwellen in einem dichten Raster auf der Oberfläche des Bauteils (1) durchgeführt wird.

3. Verfahren nach Anspruch 1 oder Anspruch 2, **dadurch gekennzeichnet, dass** die Amplitudeninformation und die Phaseninformation der mit Hilfe eines 3D-Abbildungsverfahrens, wie des 3D-SAFT-Algorithmus, gewonnenen dreidimensionalen Ortsverteilung der Streueigenschaften analysiert und ausgewertet wird.

4. Verfahren nach einem der Ansprüche 1 bis 3, **dadurch gekennzeichnet, dass** aus der dreidimensionalen Ortsverteilung der Streueigenschaften Schnitte und Projektionen bildlich dargestellt werden, wobei die Amplitudeninformation und die Phaseninformation jeweils farblich oder in Graustufen in den Darstellungen der Schnitte und Projektionen enthalten sind.

5. Verfahren nach einem der Ansprüche 1 bis 3, **dadurch gekennzeichnet, dass** die Analyse mittels vorzeichenbehafteter B-Bilder und C-Bilder durchgeführt wird.

## Claims

1. Method for detecting and classifying defects, compaction defects and gravel voids in concrete building components (1) and/or pressure defects in prestressing cuts, having the following steps:
irradiating pulsed ultrasonic waves from electrical transmission pulses at a plurality of places of the surface of the building component (1) to be examined,
receiving the reflected ultrasonic waves at a plurality of places of the surface in order to generate a plurality of electrical reception signals,
analysing and evaluating the high-frequency electrical reception signals, using the positions of the places of the irradiated ultrasonic waves and the received, reflected sound waves in order to generate a three-dimensional local distribution of the scattering properties of the building component,
**characterised in that**
the phase information of the scattering process is determined in addition to the amplitude information and assigned to the three-dimensional local distribution of the scattering properties of the building component (1), the amplitude information being used for detection of defects and the amplitude information and the phase information of the three-dimensional local distribution for classification.

2. Method according to claim 1, **characterised in that** the irradiation and reception of ultrasonic waves is implemented in a dense grid on the surface of the component (1).

3. Method according to claim 1 or claim 2, **characterised in that** the amplitude information and the phase information of the local distribution of the scattering properties which are obtained with the help of a 3D imaging method, such as the 3D-SAFT algorithm, is analysed and evaluated.

4. Method according to one of the claims 1 to 3, **characterised in that** sections and projections from the three-dimensional local distribution of the scattering properties are represented pictorially, the amplitude information and the phase information respectively being contained in colour or in steps of grey in the representations of the sections and projections.

5. Method according to one of the claims 1 to 3, **characterised in that** the analysis is implemented by means of signed B-images and C-images.

## Revendications

1. Procédé de détection et de classification de défauts, de manques de compacité et de regroupements d'agrégats dans des composants de béton (1) et/ou de fissures de compression dans les canaux de barres de précontrainte des composants de béton, comprenant les étapes suivantes :
irradiation d'ondes ultrasonores pulsées à partir d'impulsions électriques d'émission en différents points de la surface du composant à examiner (1),
réception des ondes ultrasonores réfléchies en différents points de la surface pour générer de multiples signaux électriques de réception,
analyse et évaluation des signaux électriques de réception haute fréquence par référence aux positions des points des ondes ultrasonores irradiées et des ondes sonores réfléchies pour générer une distribution tridimensionnelle des propriétés de diffusion du composant (1),
**caractérisé en ce que** :
en plus de l'information d'amplitude, l'information de phase est assignée au processus de diffusion déterminé et à la distribution tridimensionnelle des propriétés de diffusion du composant (1), dans lequel, pour détecter les imperfections, on prend en considération l'information d'amplitude et pour classifier l'information d'amplitude et l'information de phase, on prend en considération la distribution spatiale tridimensionnelle.

2. Procédé selon la revendication 1, **caractérisé en ce qu'**on effectue l'irradiation et la réception des ondes ultrasonores dans un réseau dense de la surface du composant (1).

3. Procédé selon la revendication 1 ou la revendication 2, **caractérisé en ce qu'**on effectue l'analyse et l'évaluation de l'information d'amplitude et de l'information de phase de la distribution tridimensionnelle obtenue à l'aide d'un procédé d'imagerie 3D tel qu'un algorithme 3D-SAFT des propriétés de diffusion.

4. Procédé selon l'une quelconque des revendications 1 à 3, **caractérisé en ce que**, à partir de la distribution tridimensionnelle des propriétés de diffusion, on produit des images des coupes et des projections, dans lequel l'information d'amplitude et l'information de phase, qui sont respectivement selon une information de couleur ou de niveaux de gris, sont contenues dans les descriptions des coupes et des projections.

5. Procédé selon l'une quelconque des revendications 1 à 3, **caractérisé en ce qu'**on effectue l'analyse au moyen des images B et des images C caractéristiques.
